# EUROPEAN PATENT APPLICATION

(11) **EP 4 332 114 A1**
(43) Date of publication of application: **06.03.2024**
(21) Application number: 21938540.8
(22) Date of filing: 30.04.2021
(51) Int. Cl.: C07K 14/725, C07K 19/00, C12N 15/62

(54) **METHOD FOR SCREENING ALPHA BETA-TCR HETERODIMER MUTANTS**

(71) Applicant: Liyang TCR Biotherapeutics Co., Ltd, Liyang, Jiangsu 213300 (CN)
(72) Inventor: WU, Xianhui, Shenzhen, Guangdong 518000 (CN); CHEN, Liang, Shenzhen, Guangdong 518000 (CN); TANG, Xiaoxin, Shenzhen, Guangdong 518000 (CN); LIU, Xiaobiao, Shenzhen, Guangdong 518000 (CN); LI, Hongjian, Shenzhen, Guangdong 518000 (CN)
(74) Representative: dompatent von Kreisler Selting Werner - Partnerschaft von Patent- und Rechtsanwälten mbB
(86) International application number: PCT/CN2021/091681
(87) International publication number: WO 2022/227066

(57) **Abstract**

A method for screening αβ-TCR mutants, the method comprising the construction of a phagemid. In the construction, a gene encoding an A1 polypeptide and a gene encoding B1 polypeptide are inserted into the 3' ends of genes encoding the constant regions of the α chain and the β chain of TCR respectively, and a ribosome binding site and a molecular chaperone gene are integrated into a phage vector, wherein the amino acid sequence of the A1 polypeptide is as shown in SEQ ID NO: 1 or is represented by the variant of the A1 polypeptide; the amino acid sequence of the B1 polypeptide is as shown in SEQ ID NO: 2 or is represented by the variant of the B1 polypeptide; and the variants at least retain the function of the polypeptides before mutation. The A1 peptide and the B1 peptide are used for promoting the correct pairing and folding of the αβ heterodimer TCR, so that the efficiency of displaying the correctly folded TCR on the surface of a phage is improved. The method can efficiently complete the screening of a.0 heterodimer TCR mutants.

## Description

### TECHNICAL FIELD

The present invention belongs to the field of biopharmaceutics, in particular relates to a method for screening αβ-TCR heterodimer mutants.

### BACKGROUND

In recent years, T cell receptor (TCR)-transduced T cell immunotherapy and TCR protein drugs have drawn increasing attention in the applications of antitumor therapy. Such therapies utilize TCR to specifically recognize the antigen peptide-major histocompatibility complex (pMHC) on the tumor surface. In the TCR-transduced T cell immunotherapy, TCR activates T cells to specifically kill tumor cells by specifically recognizing pMHC target on the tumor cell surfaces. It has been reported that TCRs with high affinity (*K_{D}* is generally considered to be between 0.5-10 µM) may elicit more effective antitumor responses (PNAS, 2013, 110, 6973; Immunology, 2018, 155, 123). In terms of developing TCR protein drugs, the bivalent protein drug is currently the main form, which utilize TCR with ultra-high affinity to recognize the specific pMHC target on the tumor cell surface, and T cells are simultaneously activated by the anti-CD3 antibody at the same time, thereby achieving the specific killing of tumor cells (Nat Med, 2012, 18, 980; Clin Cancer Res, 2020, 26, 5869). Regardless of the technical pathway adopted, TCR molecules with high affinity are required.

Since T cells expressing TCRs with high-affinity in the thymus are eliminated during development (Annu Rev Immunol, 2003, 21, 139), the affinity of wild-type TCR to the pMHC is low *in vivo,* with a *K_{D}* value typically only 1-500 µM (Protein Eng Des Sel, 2011, 24, 361). The improvement of the affinity of TCR to the pMHC is necessary for recognizing tumor-specific targets more effectively, thereby acting in the specific killing of tumor cells. Therefore, it is of significant importance to obtain the TCR with high affinity, high expression level, and high stability (J Biol Chem, 2005, 280, 1882; Nat Med, 2012, 18, 980; Nat Commun, 2019, 10, 4451; Nat Commun, 2020, 11, 2330; Cells 2020, 9, 1485) by *in vitro* engineering for the development of TCR therapies.

*In vitro* engineering of TCRs can be accomplished by the methods of yeast display *(*Methods Mol Biol, 2015, 1319, 95), phage display *(*J Immunol Methods, 1998, 221, 59; Nat Biotechnol 2005, 23, 349) or mammalian cell display *(*J Biol Chem, 2019, 294, 5790), and screening. The expression and display of TCR may utilize the two structures of single chain (Single-chain V_{α}-linker-V_{β}, scTCR) and double chain (Disulfide-linked V_{α}C_{α}/V_{β}C_{β}, dsTCR) (Biomol Eng, 2007, 24, 361): scTCR, as a single-chain molecule, has the advantages of small molecular weight and high solubility. In the next place, scTCR avoids the structure of heterodimeric α- and β-chains, which is less prone to generate mismatched dimers and has higher efficiency in surface displays, such as phage/yeast display. However, due to the extreme instability of scTCR *per se,* some amino acid sites are needed to be mutated prior, to improve the stability of scTCR and the efficiency of correct folding, which increases the workload before display experiments and has a rather high failure rate. dsTCR, on the other hand, does not require stability optimization, and wild-type TCR may be subject to display experiments without concerning about the adverse effects, such as structural differences and immunogenicity, resulting from the transplantation of the mutation site from scTCR to αβ-TCR heterodimers (*in vitro* refolding/lentiviral packaging). Therefore, the use of dsTCR exhibits higher efficiency during affinity maturation. Establishing a dsTCR optimization platform with high performance is thus necessary. Nevertheless, in the absence of the transmembrane region and CD3 molecules, the folding and pairing efficiency of α and β chains of TCRs is extremely low, and it is difficult to obtain the αβ-TCR correctly folded and correctly paired during phage display. It is critical during phage display that how to improve the folding efficiency of TCRs and obtain αβ-TCRs correctly paired while using dsTCR for display experiments. Many studies on the solubility and folding efficiency of TCRs are available, such as the method to generate heterodimers by using the interchain disulfide bond between the constant regions of TCR *(*Protein Eng, 2003, 16, 707; Nat Biotech, 2005, 23, 349), which is used for the development of TCR therapy. In some studies, the coiled-coil sequence is introduced to promote the refolding of dsTCR *in vitro (*Protein Sci, 1999, 8, 2418; J Immunol Methods, 2000, 236, 147), which has not been applied in phage display. Co-expression of molecular chaperones to promote protein folding are also reported in some studies, such as promoting scFv/scTCR folding using skp *(*J Immunol Methods, 2005, 306, 51).

### SUMMARY

The technical problem to be solved by the present invention is to provide a method for screening an αβ-TCR mutant, in order to overcome the shortcomings of low efficiency of the methods used to screen effective TCR mutants or to engineer TCRs in the prior art. The screening of heterodimeric αβ-TCR mutants can be accomplished efficiently using the method of the present invention.

The present invention solves the above-mentioned technical problems mainly by the following technical solutions.

The first technical solution of the present invention is: a method for screening an αβ-TCR or a mutant thereof, comprising the construction of a phagemid, in the construction: the gene encoding A1 polypeptide and the gene encoding B1 polypeptide are inserted at the 3' ends of genes encoding constant regions of the α-chain and the β-chain of a TCR respectively, and a ribosomal binding site and a molecular chaperone gene are integrated into a phage vector;
the amino acid sequence of the A1 polypeptide is as shown in SEQ ID NO: 1 or a variant thereof, the amino acid sequence of the B1 polypeptide is as shown in SEQ ID NO: 2 or a variant thereof; the variant retains at least the function of the unmutated polypeptides.

The gene encoding the constant region of TCR α chain and the gene encoding the A1 polypeptide may be linked directly, or linked by a linker. Similarly, the gene encoding the constant region of TCR β chain and the gene encoding the B1 polypeptide may be linked directly, or linked by a linker. The two linkers used above may be conventional in the art, the sequences of which may be identical or different. In the present invention, the amino acid sequences of the two linkers are identical, preferably are as shown in SEQ ID NO: 3;

The constant region of TCR α chain and the constant region of TCR β chain in the present invention may be conventional in the art, such as the constant region of human TCR. In a specific embodiment of the present invention, the amino acid sequence of the constant region of the TCR α chain is as shown in SEQ ID NO: 5. The amino acid sequence of the constant region of the TCR β chain is as shown in SEQ ID NO: 4 or SEQ ID NO: 6, or the variant thereof; the variant is preferably obtained by substitution of position 75 with A and substitution of position 89 with D in the sequence of SEQ ID NO: 6.

The introduction of the ribosome binding site and the molecular chaperone gene are used to further promote the expression and folding of the αβ-TCR heterodimer, both are preferably located at the 3' end of the gene expressing a capsid protein. As is conventional in the art, the capsid protein is used to display the target protein, and the available capsid proteins are such as pill protein, pVIII, etc. In the present invention, the pIII protein is preferred to display the target protein. Therefore, in a specific embodiment of the present invention, the ribosome binding site and the molecular chaperone gene are inserted at the 3' end of gene III.

The nucleotide sequence of the ribosome binding site may be conventional in the art, for example, its sequence is as shown in SEQ ID NO: 7.

The molecular chaperone gene may be selected as skp, FkpA, etc. skp is selected as the molecular chaperone in the present invention. The nucleotide sequence of the skp gene is preferably as shown in SEQ ID NO: 8.

The backbone of the phage vector in the present invention may be conventional in the art, such as pCANTAB5E.

Preferably, in the pCANTAB5E, a pelB signal peptide gene and/or a Myc tag gene are introduced to replace the sequence between the gIII signal and the Amber Stop Codon; more preferably, the nucleotide sequence between the pelB signal peptide gene and the Amber Stop Codon (including the both) is as shown in SEQ ID NO: 9.

myc is used in the present invention to facilitate the cleavage of trypsin to release the phage from the streptavidin magnetic beads. If triethylamine elution method is used, the myc tag may not be added.

In the present invention, the gene of the TCR α chain and the gene of the TCR β chain are preferably located in two different expression frames;
preferably, the phagemid comprises the expression element expressing following polypeptide (1) and polypeptide (2),
polypeptide (1): pelB signal peptide-variable region of TCR α chain-constant region of α chain -Linker 1-A1 peptide,
polypeptide (2): gIII signal peptide-variable region of TCR β chain -constant region of β chain-Linker 2-B1 peptide-pIII protein; wherein, the gene of B1 peptide and the gene expression pIII protein is preferably linked by Amber Stop Codon.

More preferably, the cysteine residue located behind the constant region of the TCR α chain and the constant region of the TCR β chain, and located in the N-terminal portion of the transmembrane region is subject to substitution or deletion.

pelB signal peptide used in the present invention is intended to assist in the localization of the TCR α chain to the periplasmic space, and those skilled in the art may use a similar signal peptide instead.

The second technical solution of the present invention is: a TCR obtained by screening with the method described in the first technical solution, the α chain of the TCR comprises:
(1) CDRs 1-3 or a variant thereof of the α chain variant region having the amino acid sequence as shown in SEQ ID NO: 10, wherein: the amino acid sequence of the CDR3 variant is preferably as shown in SEQ ID NO: 19;
(2) CDRs 1-3 or a variant thereof of the α chain variant region having the amino acid sequence as shown in SEQ ID NO: 14;
(3) CDRs 1-3 or a variant thereof of the α chain variant region having the amino acid sequence as shown in SEQ ID NO: 16; or
(4) CDRs 1-3 or a variant thereof of the α chain variant region having the amino acid sequence as shown in SEQ ID NO: 12, the amino acid sequence of the variant is preferably as shown in any one of SEQ ID NOs: 40-53.

The TCR as described above, wherein, the β chain preferably comprises:
(1) CDRs 1-3 or a variant thereof of a β chain variable region having the amino acid sequence as shown in SEQ ID NO: 11;
(2) CDRs 1-3 or a variant thereof of a β chain variable region having the amino acid sequence as shown in SEQ ID NO: 15; or
(3) CDRs 1-3 or a variant thereof of a β chain variable region having the amino acid sequence as shown in SEQ ID NO: 17; wherein:
   For (1): the variant is obtained by position 6 and/or position 8 of CDR3 being substituted, position 6 is preferably substituted with Y or F, position 8 is preferably substituted with R, T or Q; preferably, the sequence of the variant is as shown in any one of SEQ ID NOs: 21-24.
   For (2): the sequence of the variant is preferably as shown in any one of SEQ ID NOs: 26-30.
   For (3): a mutation occurs in 1 site, 2 sites, 3 sites or 4 sites of positions 2-6 of CDR3; when the mutation occurs in two or more sites, the two or more sites are contiguous; position 3 is preferably mutated to A, position 4 is preferably mutated to Q, position 5 is preferably mutated to G, position 6 is preferably mutated to S, R or W; preferably, the sequence of the variant is as shown in any one of SEQ ID NOs: 32-39.

The third technical solution of the present invention is: a phage vector for screening αβ-TCR heterodimers or a mutant thereof, the phage vector comprises an expression element expressing following polypeptide (1) and polypeptide (2):
polypeptide (1): pelB signal peptide-variable region of the α chain-constant region of α chain -Linker 1-A1 peptide;
polypeptide (2): gill signal peptide-variable region of the β chain-constant region of β chain-Linker 2-B1 peptide-pIII protein; wherein, the gene of B1 peptide and the gene expressing pIII protein are preferably linked by Amber Stop Codon.

As described above, wherein, the amino acid sequence of the constant region of the α chain is preferably as shown in SEQ ID NO: 5.

The amino acid sequence of the constant region of the β chain is preferably as shown in SEQ ID NO: 4 or SEQ ID NO: 6;
the amino acid sequence of the Linker 1 is preferably as shown in SEQ ID NO: 3.

The amino acid sequence of the Linker 2 is preferably as shown in SEQ ID NO: 3.

The amino acid sequence of A1 peptide is preferably as shown in SEQ ID NO: 1.

The amino acid sequence of B1 peptide is preferably as shown in SEQ ID NO: 2.

The backbone of the phage vector is preferably pCANTAB5E. During the vector construction, elements in pCANTAB5E may be deleted or adjusted in sequence as needed. During the application of the present invention, a pelB signal peptide gene and/or a Myc tag gene are preferably introduced to substitute the sequence between the gIII signal and the Amber Stop Codon. After the substitution, the nucleotide sequence between the pelB signal peptide gene and the Amber Stop Codon (including the both) is as shown in SEQ ID NO: 9.

A ribosomal binding site and a molecular chaperone gene are preferably integrated into the phage vector, such as, integrated at the 3' end of a gene expressing a capsid protein; the molecular chaperone gene is preferably a skp gene, more preferably the sequence as shown in SEQ ID NO: 8; the sequence of the ribosomal binding site is preferably as shown in SEQ ID NO: 7.

The amino acid sequence of the constant region of the α chain is preferably as shown in SEQ ID NO: 5;
the amino acid sequence of the constant region of the β chain is preferably as shown in SEQ ID NO: 4 or SEQ ID NO: 6, or a variant thereof; the variant of the sequence as shown in SEQ ID NO: 6 preferably contains mutation sites C75A and N89D.

The third technical solution of the present invention is: a use of the polypeptide as shown in SEQ ID NO: 1 or 2 in screening a TCR mutant.

During the use, the gene encoding the polypeptide is preferably placed at the 3' end of the gene of constant region of the α chain or the β chain of the TCR, respectively.

Preferably, the gene encoding the polypeptide is linked to the gene of the constant region of the α chain or the β chain of the TCR by a linker; the amino acid sequence of the linker is preferably as shown in SEQ ID NO: 3.

Based on the common knowledge in the art, each of the above preferred conditions may be combined in any way to obtain each preferred example of the present invention.

The reagents and raw materials used in the present invention are commercially available.

The positive progressive effect of the present invention is that
The present invention utilizes A1 peptide and B1 peptide to promote the correct pairing and folding of αβ heterodimeric TCR, which in turn enhances the display efficiency of correctly folded TCRs on the surface of phage. The present invention can efficiently accomplish the screening of the heterodimeric αβ-TCR mutants. The method can also be used to optimize the stability, water solubility, and affinity of TCRs.

### BRIEF DESCRIPTION OF THE DRAWINGS

FIG. 1 is the plasmid profile of the phage display vector.
FIG. 2A is a design schematic diagram of TCR gene CDS, FIG. 2B is a structure schematic diagram of TCR heterodimer.
FIG. 3 is a schematic diagram of the primer design method for library construction.
FIG. 4 is the results of ELISA assay after the second-round screening for TCR2.
FIG. 5A and FIG. 5B are the anion exchange chromatography and SDS-PAGE of TCR3-A0B1.
FIG. 6A and FIG. 6B are the gel filtration chromatography and SDS-PAGE.
FIG. 7 is the affinity test graph of TCR1-A0B4.

### DETAILED DESCRIPTION OF THE PREFERRED EMBODIMENT

The present invention is further described below in combination with specific examples. It should be understood that these examples are only intended to illustrate the present invention and are not intended to limit the scope of the present invention. Furthermore, it should be understood that after reviewing the content taught by the present invention, a person skilled in the art may make various alterations or modifications to the present invention, and these equivalent forms also fall within the scope defined by the claims appended to the present application.

### Example 1: Design and construction of the vector for phage display

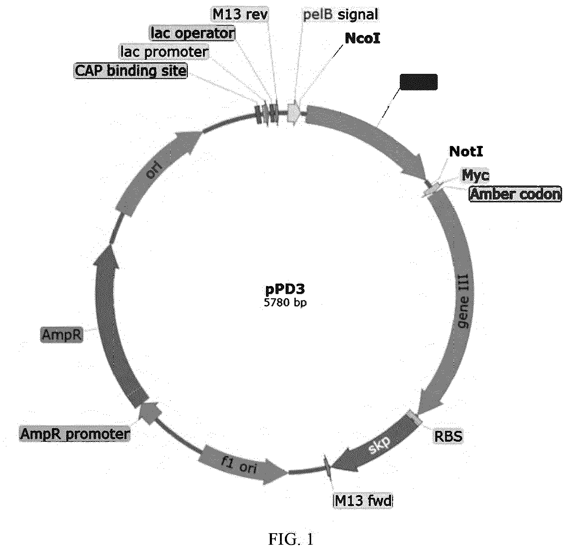

In order to display TCR in heterodimeric form on M13 phage, based on the phage display vector pCANTAB5E (purchased from Shanghai Huzhen Industrial Co., Ltd.), the main elements of the vector (ori, f1 ori, ampicillin resistance gene and gene III, etc.) were retained, and the gene sequences of pelB signal peptide, Myc tag and GFP, etc. were introduced to replace the original DNA sequence between gIII signal and Amber Stop Codon (TAG), and a ribosome binding site and molecular chaperone skp gene were added following gene III to construct the phagemid vector pPD3 (see FIG. 1 for vector profile). Restriction sites for restriction endonucleases *Nco*I and *Not*I were added at the terminus of pelB and upstream gene III, respectively, for the ligation of TCR gene to the display vector.

Nucleotide sequence between pelB and Amber Stop Codon (underlined sequences are for pelB, GFP, Myc and Amber Stop Codon in order, and bolded sequences are restriction sites of *Nco*I and *Not*I):

atgaaatacctattgcctacggcagccgctggattgttattactcgcggcccagccggccatggctctaatagtgactaata
gtgactaatagtgagtgagcaagggcgaggagctgttcaccggggtggtgcccatcctggtcgagctggacggcgacgtaaacggc
cacaagttcagcgtgtccggcgagggcgagggcgatgccacctacggcaagctgaccctgaagttcatctgcaccaccggcaagct

gcccgtgccctggcccaccctcgtgaccaccctgacctacggcgtgcagtgcttcagccgctaccccgaccacatgaagcagcacg
acttcttcaagtccgccatgcccgaaggctacgtccaggagcgcaccatcttcttcaaggacgacggcaactacaagacccgcgccg
aggtgaagttcgagggcgacaccctggtgaaccgcatcgagctgaagggcatcgacttcaaggaggacggcaacatcctggggca
caagctggagtacaactacaacagccacaacgtctatatcatggccgacaagcagaagaacggcatcaaggtgaacttcaagatccg
ccacaacatcgaggacggcagcgtgcagctcgccgaccactaccagcagaacacccccatcggcgacggccccgtgctgctgcc
cgacaaccactacctgagcacccagtccgccctgagcaaagaccccaacgagaagcgcgatcacatggtcctgctggagttcgtga
ccgccgccgggatcactctcggcatggacgagctgtacaagtaatagtgactaatagtgactaatagtgatgcggccgctggatccga
acagaaactgatcagcgaagaggatctggctgaatag (SEQ ID NO: 9)

Nucleotide sequence of the ribosome binding site and skp gene (underlined sequence):

tttgtttaactttaagaaggagatatacatatgaaaaagtggttattagctgcaggtctcggtttagcactggcaacttctgctc
aggcggctgacaaaattgcaatcgtcaacatgggcagcctgttccagcaggtagcgcagaaaaccggtgtttctaacacgctggaaa
atgagttcaaaggccgtgccagcgaactgcagcgtatggaaaccgatctgcaggctaaaatgaaaaagctgcagtccatgaaagcg
ggcagcgatcgcactaagctggaaaaagacgtgatggctcagcgccagacttttgctcagaaagcgcaggcttttgagcaggatcgc
gcacgtcgttccaacgaagaacgcggcaaactggttactcgtatccagactgctgtgaaatccgttgccaacagccaggatatcgatct
ggttgttgatgcaaacgccgttgcttacaacagcagcgatgtaaaagacatcactgccgacgtactgaaacaggttaaataa
tttgtttaactttaagaaggaga (SEQ ID NO: 7)

atgaaaaagtggttattagctgcaggtctcggtttagcactggcaacttctgctcaggcggctgacaaaattgcaatcgtca
acatgggcagcctgttccagcaggtagcgcagaaaaccggtgtttctaacacgctggaaaatgagttcaaaggccgtgccagcgaac
tgcagcgtatggaaaccgatctgcaggctaaaatgaaaaagctgcagtccatgaaagcgggcagcgatcgcactaagctggaaaaa
gacgtgatggctcagcgccagacttttgctcagaaagcgcaggcttttgagcaggatcgcgcacgtcgttccaacgaagaacgcggc
aaactggttactcgtatccagactgctgtgaaatccgttgccaacagccaggatatcgatctggttgttgatgcaaacgccgttgcttaca
acagcagcgatgtaaaagacatcactgccgacgtactgaaacaggttaaataa (SEQ ID NO: 8)

### Example 2: Construction of wild-type TCR phagemid

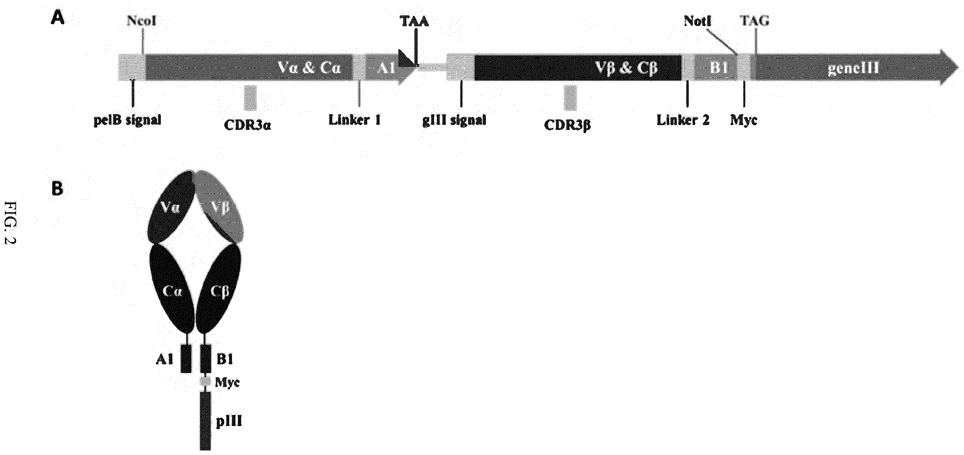

The intact heterodimeric TCR comprises an α chain variable region (Vα), an α chain constant region (Cα), a β chain variable region (Vβ) and a β chain constant region (Cβ). To express the α chain and β chain of TCR respectively, we designed and synthesize the TCR gene (the structure is shown in FIG. 2A), referring to a PhD thesis from the Department of Pharmacy at Cardiff University (Liddy N. **2013,** Molecular engineering of high affinity T-cell receptors for bispecific therapeutics). The engineered coiled-coil sequences A1 and B1 were introduced to promote the proximity of the two chains and thus folding to form TCR correctly. The α chain and β chain genes were designed to be in 2 expression frames: the A1 peptide gene was placed downstream Cα, and pelB signal peptide-Vα-Cα-Linker 1-A1 peptide were fused to express; the B1 peptide gene was placed downstream Cβ, and III signal peptide-Vβ-Cβ-Linker 2-B1 peptide-pIII protein were fused to express. The cysteine (C) that forms the disulfide bond behind the constant region (N-terminus of the transmembrane region) of both chains of the TCR was removed and no additional C was added to form the disulfide bond to reduce the misfolding caused by the dismatch of the disulfide bond; in addition, mutations of C75A and N89D were present in Cβ. The design schematic diagram of TCR gene is shown in FIG. 2A.

Amino acid sequence of A1:
STTVAQLEEKVKTLRAQNYELKSRVQRLREQVAQLAS (SEQ ID NO: 1)

Amino acid sequence of B1:
STSVDELQAEVDQLQDENYALKTKVAQLRKKVEKLSE (SEQ ID NO: 2)

Amino acid sequence of Linker 1:
GGSGGGGG (SEQ ID NO: 3)

Amino acid sequence of Linker 2:
GGSGGGGG (SEQ ID NO: 3)

Amino acid sequence of α chain constant region:
SEQ ID NO: 5

Amino acid sequence of β chain constant region (below are TRBC1 and TRBC2 in order):

EDLNKVFPPEVAVFEPSEAEISHTQKATLVCLATGFFPDHVELSWWVNGKEV
HSGVSTDPQPLKEQPALNDSRYCLSSRLRVSATFWQNPRNHFRCQVQFYGLSENDEW
TQDRAKPVTQIVSAEAWGRAD (SEQ ID NO: 4)

EDLKNVFPPEVAVFEPSEAEISHTQKATLVCLATGFYPDHVELSWWVNGKEV
HSGVSTDPQPLKEQPALNDSRYCLSSRLRVSATFWQNPRNHFRCQVQFYGLSENDEW
TQDRAKPVTQIVSAEAWGRAD (SEQ ID NO: 6)

The synthesized full-length TCR gene was double digested with *Nco*I and *Not*I, and ligated by T4 DNA ligase to the display vector which was already digested by the same restriction endonuclease. TG1 competent cells are transfected by chemical transformation. The correct phagemid was obtained by single clone sequencing analysis and used as PCR template for library construction.

Both of the two expression frames have low expression level under the regulation of the weak promoter: lac promoter and lac operator; due to the presence of the Amber Stop Codon TAG upstream gene III, the translation efficiency was reduced (translation as glutamine inefficiently in TG1 strain). The expression level of the fusion protein with β chain would be lower than that of the α chain. The low expression level of TCR gene may avoid the formation of homodimers, especially avoiding the formation of β-chain homodimer, and the β chain may have greater probability of monovalent display on the phage surface, which contributes to the formation of heterodimeric αβ-TCR. Fusion proteins are localized to the periplasmic space under the action of the pelB signal peptide and gIII signal peptide, respectively. The interaction of A1 peptide and B1 peptide promotes the binding of the α chain and the β chain, which in turn are folded to form heterodimeric αβ-TCR (shown in FIG. 2B). The TCR participated in phage packaging in the bacterial periplasmic space to form the phages that display heterodimeric αβ-TCR on surface, which were released into the culture medium.

### Example 3: Construction of mutant library

The designs of Example 1 and Example 2 are theoretically applicable to the modifications of CDR1, CDR2 and CDR3, but only CDR3 was validated. The selected 4 TCRs with 3 different targets were significantly enriched in amino acid sequences after CDR3 screening, and the mutant affinity was enhanced. Information about the targets, the germline origins and the sequences, etc. of the 4 TCRs are indicated below:

The targets and the germline origins of the 4 TCRs

| TCR | HLA | Target | TRAV | TRBV |
|---|---|---|---|---|
| TCR1 | A*02:01 | WT1(RMFPNAPYL) | TRAV1-2 | TRBV7-9 |
| TCR2 | A*02:01 | WT1(RMFPNAPYL) | TRAV39 | TRBV7-9 |
| TCR3 | A*02:01 | AFP(FMNKFIYEI) | TRAV22 | TRBV7-9 |
| TCR4 | A*02:01 | PRAME(VLDGLDVLL) | TRAV14 | TRBV19 |

TCR1-α chain variable region: GQNIDQPTEMTATEGAIVQINCTYQTSGFNGLFWYQQHAGEAPTFLSYNVL 
DGLEEKGRFSSFLSRSKGYSYLLLKELQMKDSASYLCAVSNSGAGSYQLTFGKGTK
LSVIPN (SEQ ID NO: 10)
TCR1-β chain variable region: 
DTGVSQDPRHKITKRGQNVTFRCDPISEHNRLYWYRQTLGQGPEFLTYFQN
EAQLEKSRLLSDRFSAERPKGSFSTLEIQRTEQGDSAMYLCASSLGLGEETQYFGPG
TRLLVL (SEQ ID NO: 11)
TCR2-α chain variable region: 
ELKVEQNPLFLSMQEGKNYTIYCNYSTTSDRLYWYRQDPGKSLESLFVLLS
NGAVKQEGRLMASLDTKARLSTLHITAAVHDLSATYFCGGGADGLTFGKGTHLIIQP
Y (SEQ ID NO: 12)
TCR2-β chain variable region: 
DTGVSQDPRHKITKRGQNVTFRCDPISEHNRLYWYRQTLGQGPEFLTYFQN
EAQLEKSRLLSDRFSAERPKGSFSTLEIQRTEQGDSAMYLCASSLYGGGDTQYFGPG
TRLTVL (SEQ ID NO: 13)
TCR3-α chain variable region: 
GIQVEQSPPDLILQEGANSTLRCNFSDSVNNLQWFHQNPWGQLINLFYIPSG
TKQNGRLSATTVATERYSLLYISSSQTTDSGVYFCAVEGDSNYQLIWGAGTKLIIKPD
(SEQ ID NO: 14)
TCR3-β chain variable region: 
DTGVSQDPRHKITKRGQNVTFRCDPISEHNRLYWYRQTLGQGPEFLTYFQN
EAQLEKSRLLSDRFSAERPKGSFSTLEIQRTEQGDSAMYLCASRHLYNEQFFGPGTR
LTVL (SEQ ID NO: 15)
TCR4-α chain variable region: 
AQKITQTQPGMFVQEKEAVTLDCTYDTSDQSYGLFWYKQPSSGEMIFLIYQ
GSYDEQNATEGRYSLNFQKARKSANLVISASQLGDSAMYFCAMREPRGSARQLTFG
SGTQLTVLPD (SEQ ID NO: 16)
TCR4-β chain variable region:

DGGITQSPKYLFRKEGQNVTLSCEQNLNHDAMYWYRQDPGQGLRLIYYSQI
VNDFQKGDIAEGYSVSREKKESFPLTVTSAQKNPTAFYLCASSQGIGLAGGIYEQYF
GPGTRLTVT (SEQ ID NO: 17)

The germline of the α chain constant regions of above 4 TCRs were originated from TRAC, and the germline of the β chain constant regions were originated from TRBC2 (see Example 2 for sequence details).

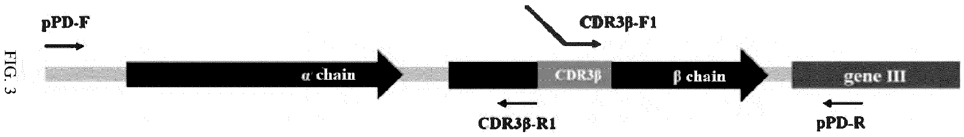

The library was constructed by introduction of the saturated mutation including 20 types of amino acid after PCR amplification with primers carrying simplex codons. The mutation primers with triplet codon NNK (5'→3', the simple base codon N represents the bases A, T, C and G; and K represents the bases G and T) were used at each amino acid site of the optimized CDR3; as the electrotransformation efficiency of the TG1 transformation competent cells was approximately 10⁸-10⁹, 4 NNKs were introduced into each mutation primer. The mutation of CDR3α was designed on the reverse primer, therefore it was MNN (5'→3', M represents the bases A and C), and the mutation of CDR3β was designed on the forward primer, therefore it was NNK (5'→3'). FIG. 3 is the design schematic diagram of the primer, wherein pPD-F and pPD-R are the universal primers designed for the vector, CDR3β-R1 is the reverse primer without mutation; CDR3β is the forward primer carrying the mutation, the 3' end of this primer may complementarily bind to the template sequence, the middle (gray bolded portion) is the NNK mutation region, and the 5' end is reversely complementary to CDR3β-R1 for overlap extension PCR amplification of the full-length TCR gene.

For TCR1, 5 mutation primers were designed for CDR3α and CDR3β according to the length of CDR. The primer names and sequences are listed in Table 1 (the lowercase letters in the primer sequences are bases that may match the template, and the bolded uppercase part is the saturated mutation region of this primer). The forward primer CDR3α-F1 was paired with the reverse primers CDR3α-R1 and CDR3α-R2, respectively, while the forward primer CDR3α-F2 was paired with the reverse primers CDR3α-R3, CDR3α-R4 and CDR3α-R5, respectively; CDR3β was used in the same way.

**Table 1 Primers for TCR1 library construction**

| **primer** | **sequence** |
|---|---|
| pPD-F | acacaggaaacagctatgac |
| pPD-R | acactgagtttcgtcaccag |
| CDR3α-F1 | ggttcctaccagctgaccttcg |
| CDR3α-R1 | aggtcagctggtaggaaccagcacc**MNNMNNMNNMNNMNN**gcacaggtaggacgcag |
| CDR3α-R2 | aggtcagctggtaggaacc**MNNMNNMNNMNNMNN**taccgcgcacaggtaggac |
| CDR3α-F2 | ttcggtaaaggtaccaaactg |
| CDR3α-R3 | agtttggtacctttaccgaaggtcagctggta**MNNMNNMNNMNN**ggagttagataccgc |
| CDR3α-R4 | agtttggtacctttaccgaaggtcag**MNNMNNMNNMNN**agcaccggagttagatacc |
| CDR3α-R5 | agtttggtacctttaccgaa**MNNMNNMNNMNN**ggaaccagcaccggag |
| CDR3β-R1 | acacaggtacatcgcagaatcg |
| CDR3β-F1 | ttctgcgatgtacctgtgt**NNKNNKNNKNNK**ggtctgggtgaagaaaccc |
| CDR3β-F2 | ttctgcgatgtacctgtgtgcatct**NNKNNKNNKNNK**ggtgaagaaacccagtac |
| CDR3β-R2 | cagggaagatgcacacaggtac |
| CDR3β-F3 | acctgtgtgcatcttccctg**NNKNNKNNKNNK**gaaacccagtactttggtc |
| CDR3β-F4 | acctgtgtgcatcttccctgggtctg**NNKNNKNNKNNK**cagtactttggtccgg |
| CDR3β-F5 | acctgtgtgcatcttccctgggtctgggtgaa**NNKNNKNNKNNK**tttggtccgggtacc |

The library was constructed using amplification methods of error prone PCR and overlap extension PCR successively to obtain the TCR gene carrying random mutations. Taking the pair of primers in FIG. 3 as an example, in the first round of PCR amplification 2 groups were divided: one group of PCR using primers pPD-F and CDR3β-R1 to amplify the fragment without mutation (about 1260 bp), and the other group using CDR3β-F1 and pPD-R to amplify the fragment carrying the mutation (about 810 bp); PCR amplification was performed using KOD FX (TOYOBO, KFX-101) in a system of 25 µL:

### The first round of PCR amplification system

| Component | Volume (µL) |
|---|---|
| 2×PCR Buffer for KOD FX | 12.5 |
| dNTPs (2.0 mM each) | 3 |
| primer F (10 µM) | 1 |
| primer R (10 µM) | 1 |
| Template (100 pg/µL) | 1 |
| KOD FX DNA Polymerase(1.0 U/µL) | 0.5 |
| ddH₂O | Up to 25 |

PCR reaction procedure: 95°C for 3 min, 95°C for 10 sec, 58°C for 30 sec, 68°C for 90 sec, 68°C for 5 min, and 4°C for holding; 1.2% agarose gel electrophoresis was performed after PCR amplification, the target band was cut out, and agarose gel recovery kit (QIAGEN, 28706) was used to recover the fragments.

The second round of PCR was performed using vector universal primers pPD-F and pPD-R, and the two small fragments purified from the first round of PCR products were used as templates to amplify the TCR gene carrying the random mutation at the mutation site of this primer (about 2070 bp); PCR amplification system of 50 µL is used:

### The second round of PCR amplification system

| Component | Volume (µL) |
|---|---|
| 2×PCR Buffer for KOD FX | 25 |
| dNTPs (2.0 mM each) | 6 |
| primer F (10 µM) | 1.5 |
| primer R (10 µM) | 1.5 |
| Template (100 pg/µL) | 3 |
| KOD FX DNA Polymerase (1.0 U/µL) | 1 |
| ddH₂O | Up to 25 |

PCR reaction procedure: 95°C for 3 min, 95°C for 10 sec, 58°C for 30 sec, 68°C for 120 sec, 68°C for 5 min, and 4°C for holding; 1% agarose gel electrophoresis was performed after PCR amplification, the target band was cut out, and agarose gel recovery kit (QIAGEN, 28706) was used to recover the fragments. The same method was used for PCR amplification with other mutation primers.

PCR amplified TCR gene and phage display vector were subject to double digestion respectively with *Nco*I-HF (NEB, R3193S) and *Not*I-HF (NEB, R3189L); digestion system of 50 µL is used:

### System of double digestion

| Component | Volume (µL) |
|---|---|
| 10×CutSmart 5 µL | 5 |
| *Nco*I-HF | 1 |
| *Not*I-HF | 1 |
| PCR fragment/display vector | 1.5 µg |
| ddH₂O | Up to 50 |

A sufficient amount of digestion system was prepared according to the number of TCR fragments and vector requirements, and cultured at 37°C for overnight; the target bands (TCR was about 1730 bp, display vector was about 4500 bp) were cut out after 1% agarose gel electrophoresis separation, and purified by agarose gel recovery kit along with concentration determination.

The vector and the fragment were ligated using T4 DNA ligase (NEB, M0202S) at a ratio of 1:3, and one ligation system was prepared for each TCR fragment; the system was cultured at 16°C for overnight.

### Ligation system

| Component | Volume (µL) |
|---|---|
| 10×Buffer | 5 |
| Display vector | 200 ng |
| PCR Fragment | 200 ng |
| T₄ DNA Ligase | 1 |
| ddH₂O | Up to 50 |

According to the different CDRs of ligation products, multiple reaction products of the same CDR were mixed, purified with the purification kit (TAKARA, 9761), eluted with sterilized deionized water, and electrotransformed into TG1 competent cells (Lucigen, 60502-2). The bacteria cells were collected after spreading the plate and culturing to complete the library construction.

### Example 4: Screening of mutant library

The mutant library was subject to screening for 2-3 rounds using the phage display method.

### 1) Phage packaging and precipitation

Library bacteria were inoculated in 50 mL 2×YT medium containing 100 µg/mL ampicillin and 2% glucose, with OD₆₀₀ between 0.05-0.08 after inoculation, and cultured at 37°C, 220 rpm until OD₆₀₀=0.4-0.5. Helper phage M13K07 was added at a ratio of 20:1 (phage:bacteria), mixed well and kept at 37°C for 30 min in stationary; the mixture was centrifuged at 3000 g for 10 min at room temperature, and then the supernatant was discarded. The pellets were resuspended with 50 mL 2×YT medium containing 100 µg/mL ampicillin and 50 µg/mL kanamycin, and cultured at 26°C, 200-220 rpm for overnight.

Bacterial cultures were centrifuged at 4°C/3000 g for 10 min. The supernatant was mixed with PEG/NaCl solution at a ratio of 4:1 and further centrifuged at 4°C/3000 g for 20 min after being kept on ice for 1 hour. The precipitates were collected and resuspended with 10 mL of PBS, centrifuged at 4°C/3000 g for 20 min. The supernatant was collected and added with 2.5 mL of PEG/NaCl solution, mixed well and kept on ice for 20-30 min; the mixture was centrifuged at 4°C/3000 g for 30 min, and the precipitates were resuspended with 1 mL of PBS to obtain the phage solution.

### 2) Phage panning

The milk solution (500 µL, 3% w/v) was added to 500 µL of phage solution described above for 1 hour blocking. Then, an appropriate amount of biotinylation-labeled pMHC solution was added, and the mixture was rotationally incubated for 1 hour at room temperature to form the phage-pMHC complex. After the addition of 50 µL streptavidin magnetic beads and 30 minutes rotational incubation at room temperature, the magnetic bead-phage-pMHC complex were further achieved. The magnetic beads were adsorbed by a magnet, and washed for 3 rounds with 1.5% Milk-PBS, 0.1% PBST, and PBS respectively. Then 0.1 mg trypsin (Sigma, T1426) (final concentration 1 mg/mL) was added and rotationally incubated for 30 min at room temperature. Via magnet adsorption, the supernatant was collected and used to infect 10 mL of pre-cultured TG1 strain (OD600=0.4-0.5). The bacteria strain was spread on the plate containing 100 µg/mL ampicillin and 2% glucose and cultured upside down at 32°C for overnight. All the bacteria pellets on the plate were collected and stored. An appropriate amount of single clones on the plate for gradient dilution counting were selected to perform sequencing analysis.

The above process was repeated for 2-3 rounds of screening according to the experimental requirements, and the 2^{nd} round or the 3^{rd} round of screening reduced the pMHC concentration appropriately to obtain higher affinity sequences according to the degree of library enrichment.

The optimization of the CDR3 of 4 different TCRs of the present invention all achieved the results of significant enrichment of amino acid sequences. CDR3α and CDR3β were optimized in TCR1, wherein CDR3α sequence was not significantly enriched, and still the mutation sites were basically present in the whole region of CDR after screening (see Table 2), which may be resulted from the amino acid mutation in this CDR contributes less to its affinity enhancement to pMHC. It can be seen from the sequencing results of the 1^{st} round screening of CDR3β that G97 and G99 were enriched (in clones CDR3β-11, 12 and 13, introduced by primer CDR3β-F3, and 4 amino acids of G97-E100 were mutated); in the sequencing results of the 2^{nd} round screening, it presents more obvious enrichment, with all L98 mutated to Y and E100 mutated to T/Q/A, while all other sites were wild-type (see Table 3).

**Table 2 Sequence analysis for library construction of TCR1 CDR3α**

| **Clone Type** | **Clone Number** | **CDR3α_{(A89-T101)}** |
|---|---|---|
| Wild type | TCR1-3α-wt | AVSNSGAGSYQLT |
| *E.coli* library clones | TCR1-3α-1 | **PDRPE**GAGSYQLT |
| | TCR1-3α-2 | AV**PLGTL**GSYQLT |
| | TCR1-3α-3 | AV**PKV**GTGSYQLT |
| | TCR1-3α-4 | AVSNS**RGKD**YQLT |
| | TCR1-3α-5 | AVSNSGA**EWAA**LT |
| | TCR1-3α-6 | AVSNSGAGS**SSRS** |
| | TCR1-3α-7 | AVSNSGAGS**NVND** |
| Clones produced by the 1^{st} round screening | TCR1-3α-8 | **S*GTK**GAGSYQLT |
| | TCR1-3α-9 | AV**CFAMP**GSYQLT |
| | TCR1-3α-10 | AV***IYHT**GSYQLT |
| | TCR1-3α-11 | AV**DDLPN**GSYQLT |
| | TCR1-3α-12 | AVSNS**FPAT**YQLT |
| | TCR1-3α-13 | AVSNS**LNLP**YQLT |
| | TCR1-3α-14 | AVSNS**L**A**PP**YQLT |
| | TCR1-3α-15 | AVSNSG**R****YQLT |
| | TCR1-3α-16 | AVSNSG**LPR**YQLT |
| | TCR1-3α-17 | AVSNSGAG**ART**LT |
| | TCR1-3α-18 | AVSNSGA**LDHS**LT |
| | TCR1-3α-19 | AVSNSGA**QVAR**LT |
| | TCR1-3α-20 | AVSNSG**G**GS**NGWA** |
| Clones produced by the 2^{nd} round screening | TCR1-3α-21 | AV**RCTQP**GSYQLT |
| | TCR1-3α-22 | AVSNS**FVEL**YQLT |
| | TCR1-3α-23 | AVSNS**QRV**LYQLT |
| | TCR1-3α-24 | AVSNSGA**S*CV**LT |
| | TCR1-3α-25 | AVSNSGA**YVFP**LT |
| | TCR1-3α-26 | AVSNSGAGS**RRWE** |
| Note: the nucleotide sequence corresponding to * is Amber Stop Codon TAG | | |

**Table 3 Sequence analysis for library construction of TCR1 CDR3β**

| **Clone Type** | **Clone Number** | **CDR3β**_{(A93-Y104)} |
|---|---|---|
| Wild type | TCR1-3β-wt | ASSLGLGEETQY |
| *E. coli* library clones | TCR1-3β-1 | **PQEP**GLGEETQY |
| | TCR1-3β-2 | **TDM**LGLGEETQY |
| | TCR1-3β-3 | A**P*T**GLGEETQY |
| | TCR1-3β-4 | AS**PRAL**GEETQY |
| | TCR1-3β-5 | ASSL**PTK**EETQY |
| | TCR1-3β-6 | ASSLGT**LVYL**QY |
| | TCR1-3β-7 | ASSLGL**Q*LA**QY |
| Clones produced by the 1^{st} round screening | TCR1-3β-8 | AS**GMVS**GEETQY |
| | TCR1-3β-9 | AS***TYA**GEETQY |
| | TCR1-3β-10 | ASSL**RELI**ETQY |
| | TCR1-3β-11 | ASSLG**M**G**R**ETQY |
| | TCR1-3β-12 | ASSLG**F**G**H**ETQY |
| | TCR1-3β-13 | ASSLG**Y**G**S**ETQY |
| | TCR1-3β-14 | ASSLGL**GEET**QY |
| | TCR1-3β-15 | ASSLGL**PPGA**QY |
| | TCR1-3β-16 | ASSLGL**WRRR**QY |
| | TCR1-3β-17 | ASSLGLG**R**E**I**QY |
| | TCR1-3β-18 | ASSLGLG**YDY**QY |
| | TCR1-3β-19 | ASSLGLG**CLV**QY |
| Clones produced by the 2^{nd} round screening | TCR1-3β-20 | ASSLG**Y**G**T**ETQY |
| | TCR1-3β-21 | ASSLG**Y**G**T**ETQY |
| | TCR1-3β-22 | ASSLG**Y**G**T**ETQY |
| | TCR1-3β-23 | ASSLG**Y**G**T**ETQY |
| | TCR1-3β-24 | ASSLG**Y**G**T**ETQY |
| | TCR1-3β-25 | ASSLG**Y**G**T**ETQY |
| | TCR1-3β-26 | ASSLG**Y**G**T**ETQY |
| | TCR1-3β-27 | ASSLG**Y**G**T**ETQY |
| | TCR1-3β-28 | ASSLG**Y**G**T**ETQY |
| | TCR1-3β-29 | ASSLG**Y**G**T**ETQY |
| | TCR1-3β-30 | ASSLG**Y**G**T**ETQY |
| | TCR1-3β-31 | ASSLG**Y**G**T**ETQY |
| | TCR1-3β-32 | ASSLG**Y**G**T**ETQY |
| | TCR1-3β-33 | ASSLG**Y**G**T**ETQY |
| | TCR1-3β-34 | ASSLG**Y**G**Q**ETQY |
| | TCR1-3β-35 | ASSLG**Y**G**A**ETQY |
| Note: the nucleotide sequence corresponding to * is Amber Stop Codon TAG | | |

The sequence enrichment of CDR3α of TCR2 was obvious after two rounds of phage display library screening: concentrated in the first four amino acid sites G90 for G/A, G91 mainly for G/A/V, G92 remained wild type, A93 mainly mutated for V/E, etc. of CDR3α. The sequencing analysis results are shown in Table 4.

**Table 4 Sequence analysis for library construction of TCR2 CDR3α**

| **Clone Type** | **Clone Number** | **CDR3α_{(G90-T97)}** |
|---|---|---|
| Wild type | TCR2-3α-wt | GGGADGLT |
| *E. coli* library clones | TCR2-3α-1 | **RMIV**DGLT |
| | TCR2-3α-2 | **KN**G**L**DGLT |
| | TCR2-3α-3 | **ALW**ADGLT |
| | TCR2-3α-4 | GGGA**AVDG** |
| | TCR2-3α-5 | GGGA**MPPG** |
| | TCR2-3α-6 | GGGA**HVKW** |
| | TCR2-3α-7 | GGGAD**IWS** |
| Clones produced by the 1^{st} round screening | TCR2-3α-8 | **Y**G***L**DGLT |
| | TCR2-3α-9 | ***PAE**DGLT |
| | TCR2-3α-10 | G**V**G**T**DGLT |
| | TCR2-3α-11 | GG**AHPS**LT |
| | TCR2-3α-12 | GG**A**A**VW**LT |
| | TCR2-3α-13 | GG**YVKL**LT |
| | TCR2-3α-14 | GG***LRW**LT |
| | TCR2-3α-15 | GGGA**EIVA** |
| Clones produced by the 2^{nd} round screening | TCR2-3α-16 | **QKAE**DGLT |
| | TCR2-3α-17 | **AL**G**I**DGLT |
| | TCR2-3α-18 | **AA**G**V**DGLT |
| | TCR2-3α-19 | **AA**G**V**DGLT |
| | TCR2-3α-20 | **A**GG**V**DGLT |
| | TCR2-3α-21 | G**V**G**T**DGLT |
| | TCR2-3α-22 | G**V**G**S**DGLT |
| | TCR2-3α-23 | G**I**G**D**DGLT |
| | TCR2-3α-24 | G**V**G**E**DGLT |
| | TCR2-3α-25 | G**V**G**E**DGLT |
| | TCR2-3α-26 | GG**DIT***LT |
| | TCR2-3α-27 | GGGA**GWM*** |
| | TCR2-3α-28 | GGGAD**HFC** |
| Note: the nucleotide sequence corresponding to * is Amber Stop Codon TAG | | |

CDR3β of TCR3 was significantly enriched, and the sequencing results of the 2^{nd} round screening show that the mutation sites were mainly at 5 amino acid sites from Q95 to N99, and the results are shown in Table 5.

**Table 5 Sequence analysis for library construction of TCR3 CDR3β**

| **Clone Type** | **Clone Number** | **CDR3β_{(A93-FlO2)}** |
|---|---|---|
| Wild type | TCR3-3β-wt | ASRHLYNEQF |
| *E. coli* library clones | TCR3-3β-1 | **TLQF**LYNEQF |
| | TCR3-3β-2 | **EGKW**LYNEQF |
| | TCR3-3[3-3 | **PHFL**LYNEQF |
| | TCR3-3[3-4 | **RTVE**LYNEQF |
| | TCR3-3[3-5 | AS**VQTT**NEQF |
| | TCR3-3β-6 | AS**PYD***NEQF |
| | TCR3-3β-7 | ASRH**SR*C**QF |
| | TCR3-3β-8 | ASRHLY**FDNG** |
| Clones produced by the 1^{st} round screening | TCR3-3β-9 | **C**S**NT**LYNEQF |
| | TCR3-3β-10 | **R**P**LYNEQF |
| | TCR3-3β-11 | **ER**R**P**LYNEQF |
| | TCR3-3β-12 | AS**WNVS**NEQF |
| | TCR3-3β-13 | AS***L**L**C**NEQF |
| | TCR3-3β-14 | AS**YVVS**NEQF |
| | TCR3-3β-15 | AS**HPAR**NEQF |
| | TCR3-3β-16 | AS**ANAV**NEQF |
| | TCR3-3β-17 | AS**GRVR**NEQF |
| | TCR3-3β-18 | ASRHLY**SSGR** |
| | TCR3-3β-19 | ASRHLY**PLYA** |
| Clones produced by the 2^{nd} round screening | TCR3-3β-20 | AS**QSW**YNEQF |
| | TCR3-3β-21 | AS**QRW**YNEQF |
| | TCR3-3β-22 | ASR**REF**NEQF |
| | TCR3-3β-23 | ASR**REF**NEQF |
| | TCR3-3β-24 | ASR**REF**NEQF |
| | TCR3-3β-25 | ASR**REF**NEQF |
| | TCR3-3β-26 | ASR**RSF**NEQF |
| | TCR3-3β-27 | ASR**L**L**F**NEQF |
| | TCR3-3β-28 | ASRH**EFD**EQF |
| | TCR3-3β-29 | ASRH**EFD**EQF |
| | TCR3-3β-30 | ASRH**EFD**EQF |
| | TCR3-3β-31 | ASRHLY**PGAL** |
| Note: the nucleotide sequence corresponding to * is Amber Stop Codon TAG | | |

CDR3β of TCR4 was significantly enriched: it can be seen from the sequencing results of the 2^{nd} round screening that S94 is S/A, Q95 is I/V/L, G96 is T, I97 is R, etc.; the results are shown in Table 6.

**Table 6 Sequence analysis for library construction of TCR4 CDR3β**

| **Clone Type** | **Clone Number** | **CDR3β_{(A92-F107)}** |
|---|---|---|
| Wild type | TCR4-3β-wt | ASSQGIGLAGGIYEQY |
| *E. coli* library clones | TCR4-3β-1 | **NLDD**GIGLAGGIYEQY |
| | TCR4-3β-2 | **SCWI**GIGLAGGIYEQY |
| | TCR4-3β-3 | ***PFL**GIGLAGGIYEQY |
| | TCR4-3β-4 | AS**GHR**IGLAGGIYEQY |
| | TCR4-3β-5 | ASSQ**CGV**LAGGIYEQY |
| | TCR4-3β-6 | ASSQGIGL**PE**G**R**YEQY |
| | TCR4-3β-7 | ASSQGIGLAGGI**ERSL** |
| | TCR4-3β-8 | ASSQGIGLAGGI**RSKI** |
| Clones produced by the 1^{st} round screening | TCR4-3β-9 | AS**ALTR**GLAGGIYEQY |
| | TCR4-3β-10 | AS**ALSY**GLAGGIYEQY |
| | TCR4-3β-11 | AS**AITD**GLAGGIYEQY |
| | TCR4-3β-12 | ASS**VTY**GLAGGIYEQY |
| | TCR4-3β-13 | ASS**ITS**GLAGGIYEQY |
| | TCR4-3β-14 | ASS**VTL**GLAGGIYEQY |
| | TCR4-3β-15 | ASS**LTY**GLAGGIYEQY |
| | TCR4-3β-16 | ASS**MTR**GLAGGIYEQY |
| | TCR4-3β-17 | ASS**VTM**GLAGGIYEQY |
| | TCR4-3β-18 | ASS**ITR**GLAGGIYEQY |
| | TCR4-3β-19 | ASSQ**SR**GLAGGIYEQY |
| | TCR4-3β-20 | ASSQ**TSKC**A GGIYEQY |
| Clones produced by the 2^{nd} round screening | TCR4-3β-21 | AS**AITR**GLAGGIYEQY |
| | TCR4-3β-22 | AS**AVTR**GLAGGIYEQY |
| | TCR4-3β-23 | AS**ALTS**GLAGGIYEQY |
| | TCR4-3β-24 | AS**ALTW**GLAGGIYEQY |
| | TCR4-3β-25 | ASS**LT**IGLAGGIYEQY |
| | TCR4-3β-26 | ASS**ITR**GLAGGIYEQY |
| | TCR4-3β-27 | ASS**ITR**GLAGGIYEQY |
| | TCR4-3β-28 | ASS**VTR**GLAGGIYEQY |
| | TCR4-3β-29 | ASS**LTR**GLAGGIYEQY |
| | TCR4-3β-30 | ASS**LTR**GLAGGIYEQY |
| | TCR4-3β-31 | ASS**LTH**GLAGGIYEQY |
| | TCR4-3β-32 | ASS**LTT**GLAGGIYEQY |
| | TCR4-3β-33 | ASS**LTS**GLAGGIYEQY |
| Note: the nucleotide sequence corresponding to * is Amber Stop Codon TAG | | |

### 3) ELISA assay of phage

For the successfully screened CDRs, single clones from the small plates spread with dilution were selected and inoculated into 150 mL 2×YT medium (containing 100 µg/mL ampicillin and 2% glucose) and cultured at 37°C, 200-220 rpm for overnight. 2-5 µL of bacteria solution of each clone was pipetted into a new 96-well plate containing 200 µL of the same medium and cultured at 37°C, 200-220 rpm for 3 hours. Then 50 µL of helper phage with sufficient titer was added into each well and incubated at 37°C, 200-220 rpm for 1 hour. The supernatant was discarded after the mixture was centrifuged with low speed for 10 minutes at room temperature, and the pellets were resuspended with 200 µL medium (2×YT medium with 100 µg/mL ampicillin + 50 µg/mL kanamycin), and cultured at 26°C, 200-220 rpm for overnight.

0.5 µg of streptavidin was added to each well of a 96-well ELISA plate. The ELISA plate was kept at 4°C in the refrigerator in stationary for overnight, then washed twice with PBS. 0.5 µg of biotin-labeled pMHC was added to each well, and reacted for 30 minutes at room temperature. 400 µL of 3% milk PBS solution was added for blocking at room temperature for 1 hour.

After 100 µL of fresh phage supernatant and an identical volume of milk solution were added for 1 hour blocking at room temperature, 100 µL of the solution was collected and added to the ELSA plate which was washed with PBS for 3 rounds. The reaction was continued at room temperature for 1 hour.

The ELISA plate was washed with 0.1% PBST for 3 rounds, 100 µL of diluted M13-HRP antibody (Sinobiological, Cat. No. 11973-MM05T-H, with a dilution ratio of 1:10000) was added, and reacted for 30 minutes at room temperature. The ELISA plate was washed again with 0.1% PBST for 5 rounds, and 50 µL color development solution was added to each well to develop color for 90 seconds. Then the reaction was terminated immediately, followed by the determination of absorption value (OD₄₅₀) via the microplate reader.

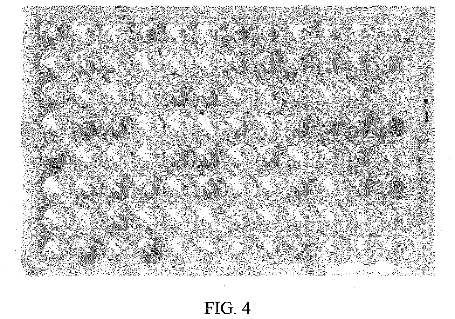

The result of TCR2 is taken as an example for phage ELISA assay. FIG. 4 is the photograph of the ELISA plate after the 2^{nd} round screening of single clone ELISA assay for TCR2, and Table 7 shows the OD₄₅₀ readings of the plate by the microplate reader.

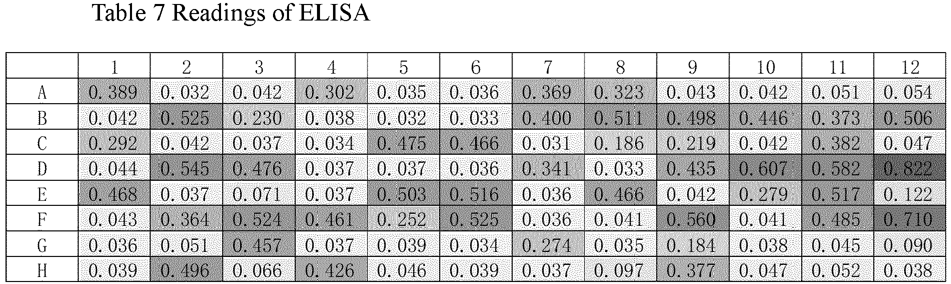

The single clones with OD₄₅₀ values greater than 0.2 in Table 7 were selected and sent to the sequencing company for sequencing analysis. The corresponding mutant sequences were obtained according to the sequencing results (see Table 8).

**Table 8 ELISA sequencing results of TCR2 CDR3α**

| Clone Number | **CDR3α_{(G90-T97)}** | Sequence Frequency | SEQ ID NO: |
|---|---|---|---|
| TCR2-3α-wt | GGGADGLT | | |
| TCR2-3α-ELISA-1 | G**I**G**D**DGLT | 1 | 40 |
| TCR2-3α-ELISA-2 | G**A**G**T**DGLT | 1 | 41 |
| TCR2-3α-ELISA-3 | G**A**G**V**DGLT | 1 | 42 |
| TCR2-3α-ELISA-4 | G**V**G**V**DGLT | 4 | 43 |
| TCR2-3α-ELISA-5 | G**V**G**D**DGLT | 1 | 44 |
| TCR2-3α-ELISA-6 | **A**GG**V**DGLT | 4 | 45 |
| TCR2-3α-ELISA-7 | **AA**G**V**DGLT | 8 | 46 |
| TCR2-3α-ELISA-8 | **AA**G**T**DGLT | 2 | 47 |
| TCR2-3α-ELISA-9 | **AV**G**D**DGLT | 3 | 48 |
| TCR2-3α-ELISA-10 | **AI**G**D**DGLT | 2 | 49 |
| TCR2-3α-ELISA-11 | **AE**G**V**DGLT | 2 | 50 |
| TCR2-3α-ELISA-12 | **SV**G**D**DGLT | 1 | 51 |
| TCR2-3α-ELISA-13 | **AV**G**V**DGLT | 1 | 52 |
| TCR2-3α-ELISA-14 | **AV**G**T**DGLT | 1 | 53 |

After the mutant library screening and ELISA assay for TCR1, TCR3 and TCR4, the obtained mutant sequences are listed in Table 9 ("A" represents α chain, "B" represents β chain, and the numbers with different "A" or "B" indicate the "α chain" or "β chain" containing different mutations; additionally, the heterodimer consisting of TCR α chain and TCR β chain is abbreviated as AmBn in the following examples; wherein m is an integer from 0 to N and n is an integer from 0 to N).

**Table 9 Mutant sequences of TCR1, TCR3 and TCR4**

| **TCR** | **Sequence Number** | **CDR3α** | **CDR3β** | **SEQ ID NO:** |
|---|---|---|---|---|
| TCR1 | A0 | AVSNSGAGSYQLT | | 18 |
| | B0 | | ASSLGLGEETQY | 20 |
| | A1 | AVSNS**LD**G**Y**YQLT | | 19 |
| | B1 | | ASSLG**Y**G**R**ETQY | 21 |
| | B2 | | ASSLG**F**G**R**ETQY | 22 |
| | B3 | | ASSLG**Y**G**T**ETQY | 23 |
| | B4 | | ASSLG**Y**G**Q**ETQY | 24 |
| TCR3 | B0 | | ASRHLYNEQF | 25 |
| | B1 | | AS**QSW**YNEQF | 26 |
| | B2 | | AS**QRW**YNEQF | 27 |
| | B3 | | ASR**REF**NEQF | 28 |
| | B4 | | ASRH**EFD**EQF | 29 |
| | B5 | | ASR**L**L**F**NEQF | 30 |
| TCR4 | B0 | | ASSQGIGLAGGIYEQY | 31 |
| | B1 | | ASS**LT**IGLAGGIYEQY | 32 |
| | B2 | | ASS**LTR**GLAGGIYEQY | 33 |
| | B3 | | ASS**LTS**GLAGGIYEQY | 34 |
| | B4 | | AS**ALTW**GLAGGIYEQY | 35 |
| | B5 | | ASS**L**GIGLAGGIYEQY | 36 |
| | B6 | | ASSQ**T**IGLAGGIYEQY | 37 |
| | B7 | | ASSQG**R**GLAGGIYEQY | 38 |
| | B8 | | ASSQG**S**GLAGGIYEQY | 39 |

### Example 5: Inclusion body expression, TCR in vitro refolding and affinity test

### 1) Inclusion body expression

Some of the mutant sequences obtained in Example 3 and Example 4 were selected, whose amino acid mutations were transplanted into A0 or B0 of the TCR. The mutants were transformed into *E. coli* BL21 (DE3) respectively, and single clones were selected and inoculated into LB medium, cultured at 37°C, 250 rpm with shaking until OD₆₀₀ = 0.6-0.8. IPTG was then added to a final concentration of 0.8 mM and the bacteria were continued to be cultured at 37°C for 3 h. The bacteria pellets were collected by centrifugation at 6000 rpm for 10 min and stored at -20°C.

The bacteria pellets were resuspended with lysis solution (PBS containing 0.5% TritonX-100), extracted by ultrasonication, and then centrifuged at a high speed of 12000 rpm for 20 min. The supernatant was discarded, and the precipitates were resuspended with lysis solution until no pellet was visible, then were subject to centrifugation at high speed for 10 min. The above operation was repeated for 2-3 rounds. The precipitates were dissolved with 6 M guanidine hydrochloride solution, centrifuged at high speed for 10 min to collect the supernatant, which referred as the purified inclusion body. 1 µL of the supernatant was subject to perform SDS-PAGE to identify the purity of inclusion body. The inclusion body was quantified, separated, and froze for storage at -80°C.

### 2) TCR in vitro refolding

15 mg inclusion bodies of TCR α chain and 10 mg inclusion bodies TCR β chain were diluted in 5 mL 6M guanidine hydrochloride solution, respectively. The TCR α chain and TCR β chain were slowly added sequentially to the pre-cooled refolding buffer (Science 1996, 274, 209*;* J. Mol. Biol. 1999, 285, 1831; Protein Eng. 2003, 16, 707) and mixed with continuous stirring for 30 min at 4°C. The mixture was then put into the dialysis bag and dialyzed in 10 times the volume of pre-cooled deionized water with stirring for 8-12 hours. The dialysis was performed in pre-cooled dialysis solution (pH 8.1, 20 mM Tris-HCl) at 4°C for 8 hours, and repeated for 2-3 rounds.

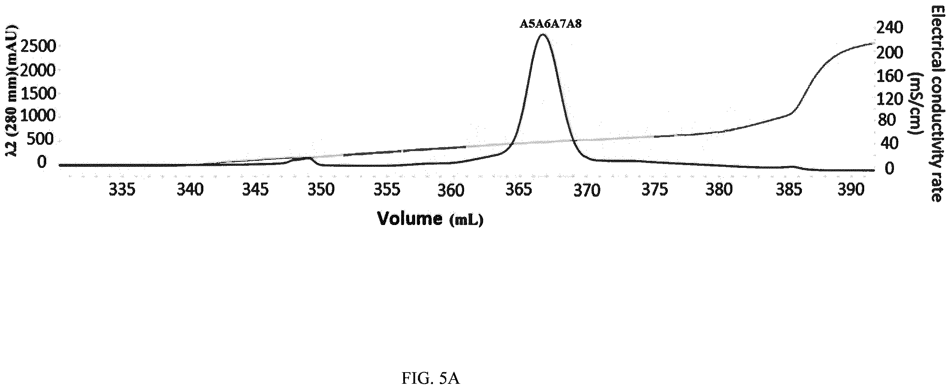

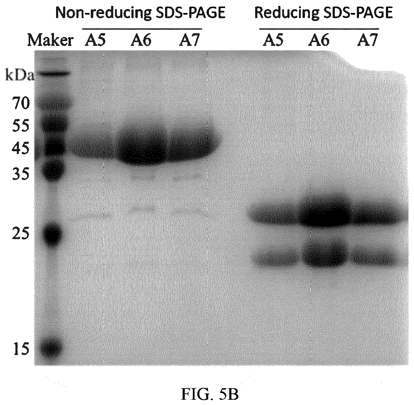

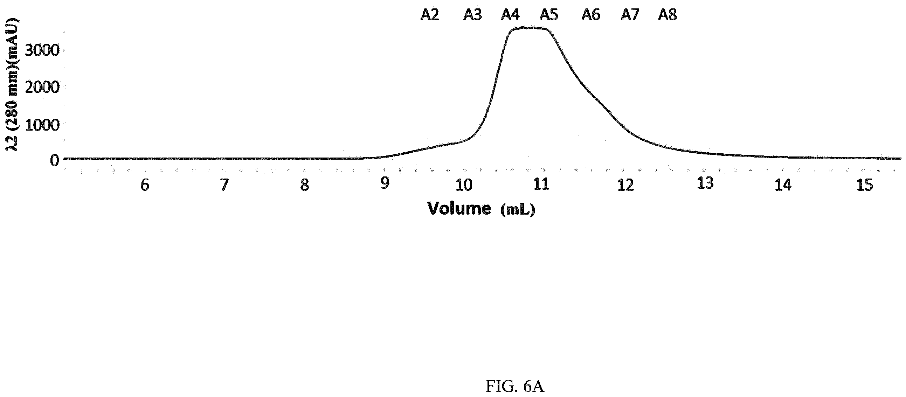

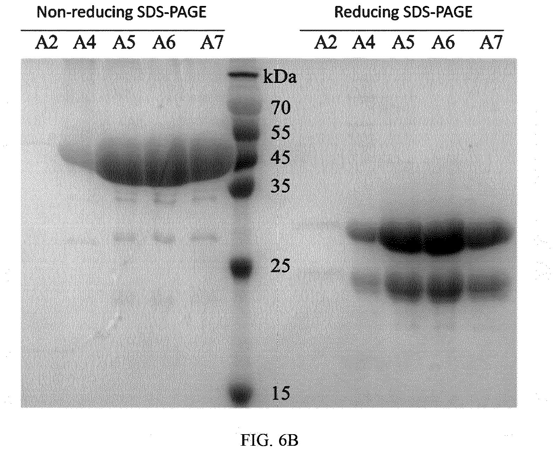

The solution was poured out from dialysis bag and centrifuged at high speed for 10 min to remove precipitation and air bubbles, and subject to anion exchange chromatography with HiTrap Q HP (5 mL). The solution was linearly eluted with 0-2 M NaCl, 20 mM Tris, pH 8.1. The elution peaks were collected for non-reducing SDS-PAGE, and the elution peaks containing the target protein fractions were combined and concentrated. The concentrated protein samples were subject to size-exclusion chromatography with superdex 75 10/300. Taking A0B1 of TCR3 as an example, the results of protein purification and SDS-PAGE are shown in FIG. 5A, FIG. 5B, FIG. 6A and FIG. 6B.

### 3) Affinity test

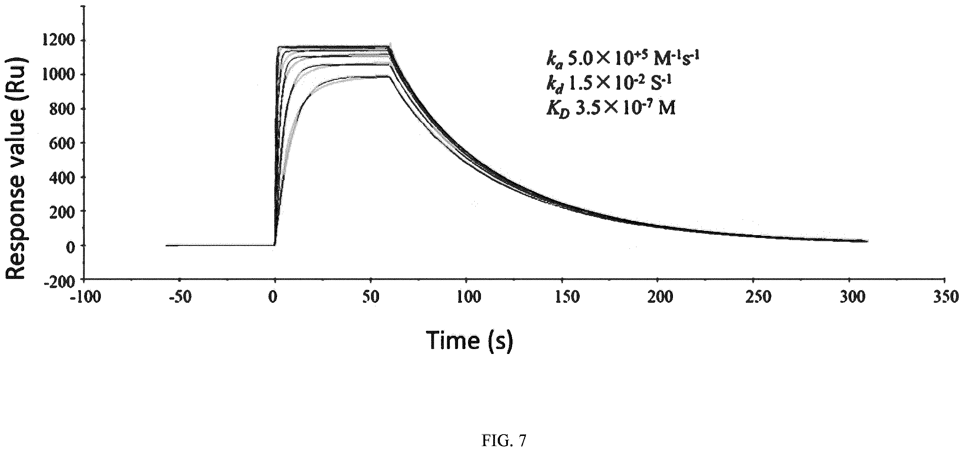

Biacore is an instrument for affinity based using surface plasmon resonance (SPR) technique. The binding and dissociation constants were calculated by monitoring the change of SPR angle and analysis. In this experiment, biotinylated pMHC was coupled on the biosensor surface and its binding and dissociation constants with different TCRs were detected to calculate the *K_{D}* values. Biacore T200 was used to perform the affinity test for the purified TCR. Taking A0B4 of TCR1 as an example, its affinity with HLA-A*02:01/WT1 (peptide RMFPNAPYL) was tested, the details are shown in FIG. 7.

Table 10 summarizes the affinity between each refolding-validated TCR mutant and its corresponding pMHC. It can be seen that the mutants obtained by screening with the designed method for heterodimeric display method have more than 2-fold improvement in affinity compared with the wild-type TCR.

**Table 10 Affinity of some mutants**

| **TCR** | **Validation Number** | **CDR3α** | **CDR3β** | ***K*_{D}(M)** |
|---|---|---|---|---|
| TCR1 | A0B0 | AVSNSGAGSYQLT | ASSLGLGEETQY | 5.5E-05 |
| | A0B3 | AVSNSGAGSYQLT | ASSLG**Y**G**T**ETQY | 6.5E-08 |
| | A0B4 | AVSNSGAGSYQLT | ASSLG**Y**G**Q**ETQY | 3.5E-07 |
| TCR2 | A0B0 | GGGADGLT | ASSLYGGGDT | 6.9E-05 |
| | A8B0 | **AE**G**V**DGLT | ASSLYGGGDT | 1.5E-05 |
| | A9B0 | G**V**G**D**DGLT | ASSLYGGGDT | 2.5E-05 |
| TCR3 | A0B0 | AVEGDSNYQLI | ASRHLYNEQF | 6.1E-05 |
| | A0B1 | AVEGDSNYQLI | AS**QSW**YNEQF | 1.7E-05 |
| | A0B2 | AVEGDSNYQLI | AS**QRW**YNEQF | 1.5E-05 |
| | A0B3 | AVEGDSNYQLI | ASR**REF**NEQF | 1.9E-05 |
| | A0B4 | AVEGDSNYQLI | ASRH**EFD**EQF | 1.3E-05 |
| | A0B5 | AVEGDSNYQLI | ASR**L**L**F**NEQF | 3.1E-05 |

The present invention creatively places A1 peptide coding sequence at the 3' end of TCR α chain coding sequence to encode fusion protein: α chain-A1 peptide; places B1 peptide coding sequence at the 3' end of TCR β chain coding sequence (expression with gene III, with Amber Stop Codon between the two, by fusion) to encode fusion protein: β chain-B1 peptide-pIII; the expression level of α chain-A1 peptide fusion protein is much higher than that of β chain-B1 peptide-pIII fusion protein. The expression level of B1 peptide is extremely low, reducing the possibility of forming a β chain-B1 peptide-pIII fusion protein homodimer, and allowing more TCR monovalent display on the phage surface at the same time. If the α chain-A1 peptide forms a homodimer due to the formation of A1 peptide dimer, the dimer does not link to phage pill protein and therefore cannot display on phage surface. The αβ heterodimeric TCR forms only when A1 and B1 form a heterodimer to promote the pairing of α chain and β chain of the TCR. skp molecular chaperone further promotes the folding of both chains of the TCR to improve the formation rate of the αβ heterodimeric TCR. After α chain-A1 peptide and β chain-B1 peptide form the αβ heterodimeric TCR, it will display on the phage surface by the pIII protein expressed by fusion. The present invention utilizes A1 peptide and B1 peptide, with the assistance of skp, to promote the correct pairing and folding of αβ heterodimeric TCR, which in turn enhances the display efficiency of correctly folded TCRs on the phage surface. The experimental results show that the present invention may efficiently accomplish the screening of αβ heterodimeric TCR mutants. The method may also be used to optimize the stability, water solubility, and affinity of TCRs.

Although the above describes specific embodiments of the present invention, it should be understood by those skilled in the art that these are merely illustrative examples and that a variety of changes or modifications can be made to these embodiments without departing from the principles and substance of the present invention. Therefore, the scope of protection of the present disclosure is limited by the appended claims.

## Claims

1. A method for screening an αβ-TCR or a mutant thereof, comprising the construction of a phagemid, wherein, in the construction: a gene encoding A1 polypeptide and a gene encoding B1 polypeptide are inserted at the 3' ends of genes encoding constant regions of the α chain and the β chain of a TCR, respectively, and a ribosomal binding site and a molecular chaperone gene are integrated into a phage vector;
the amino acid sequence of the A1 polypeptide is as shown in SEQ ID NO: 1 or a variant thereof, the amino acid sequence of the B1 polypeptide is as shown in SEQ ID NO: 2 or a variant thereof; the variant retains at least the function of the unmutated polypeptide.

2. The method according to claim 1, wherein, the constant region of the TCR α chain and the gene encoding the A1 polypeptide are linked by Linker 1; the amino acid sequence of Linker 1 is preferably as shown in SEQ ID NO: 3;
and/or, the constant region of the TCR β chain and the gene encoding the B1 polypeptide are linked by Linker 2; the amino acid sequence of Linker 2 is preferably as shown in SEQ ID NO: 3;
and/or, the amino acid sequence of the constant region of the TCR α chain is as shown in SEQ ID NO: 5;
and/or, the amino acid sequence of the constant region of the TCR β chain is as shown as SEQ ID NO: 4 or SEQ ID NO: 6, or a variant thereof; the variant of the sequence as shown in SEQ ID NO: 6 preferably comprises mutation sites C75A and N89D.

3. The method according to claims 1 or 2, wherein, the ribosome binding site and the molecular chaperone gene are located at the 3' end of the gene expressing a capsid protein, the capsid protein is preferably a pIII protein;
and/or, the nucleotide sequence of the ribosomal binding site is as shown in SEQ ID NO: 7;
and/or, the molecular chaperone gene is skp gene, the nucleotide sequence of the skp gene is preferably as shown in SEQ ID NO: 8.

4. The method according to claims 1 or 2, wherein, the backbone of the phage vector is pCANTAB5E;
preferably, in the pCANTAB5E, the pelB signal peptide gene and/or the Myc tag gene are introduced to replace the sequence between the gIII signal and the Amber Stop Codon;
more preferably, the nucleotide sequence between the pelB signal peptide gene and the Amber Stop Codon (both are comprised) is as shown in SEQ ID NO: 9.

5. The method according to claims 1 or 2, wherein, the gene of the TCR α chain and the gene of the TCR β chain are located in two different expression frames;
preferably, the phagemid comprises an expression element expressing following polypeptide (1) and polypeptide (2),
polypeptide (1): pelB signal peptide-variable region of TCR α chain (Vα)-constant region of α chain (Cα)-Linker 1-A1 peptide,
polypeptide (2): gill signal peptide-variable region of TCR β chain (Vβ)-constant region of β chain (Cβ)-Linker 2-B1 peptide-pIII protein;
more preferably, the cysteine residue located behind the constant region of the TCR α chain and the constant region of the TCR β chain, and located in N-terminal portion of the transmembrane region is subject to substitution or deletion.

6. A TCR obtained by screening with the method according to any one of claims 1-5, wherein, the α chain of the TCR comprises:
(1) CDRs 1-3 or a variant thereof of the α chain variant region having the amino acid sequence as shown in SEQ ID NO: 10, wherein: the amino acid sequence of the CDR3 variant is preferably as shown in SEQ ID NO: 19;
(2) CDRs 1-3 or a variant thereof of the α chain variant region having the amino acid sequence as shown in SEQ ID NO: 14; or
(3) CDRs 1-3 or a variant thereof of the α chain variant region having the amino acid sequence as shown in SEQ ID NO: 16.

7. The TCR according to claim 6, wherein, the β chain of the TCR comprises:
(1) CDRs 1-3 or a variant thereof of the β chain variable region having the amino acid sequence as shown in SEQ ID NO: 11, wherein: the variant is obtained by position 6 and/or position 8 of CDR3 being substituted, position 6 is preferably substituted with Y or F, position 8 is preferably substituted with R, T or Q; preferably, the sequence of the variant is as shown in any one of SEQ ID NOs: 21-24;
(2) CDRs 1-3 or a variant thereof of the β chain variable region having the amino acid sequence as shown in SEQ ID NO: 15, wherein, the sequence of the variant is preferably as shown in any one of SEQ ID NOs: 26-30;
or (3) CDRs 1-3 or a variant thereof of the β chain variable region having the amino acid sequence as shown in SEQ ID NO: 17, wherein, a mutation occurs in 1 site, 2 sites, 3 sites or 4 sites of positions 2-6 of CDR3; when the mutation occurs in two or more sites, the two or more sites are contiguous; position 3 is preferably mutated to A, position 4 is preferably mutated to Q, position 5 is preferably mutated to G, position 6 is preferably mutated to S, R or W; preferably, the sequence of the variant is as shown in any one of SEQ ID NOs: 32-39.

8. A phage vector for screening an αβ-TCR heterodimer or a mutant thereof, wherein, the phage vector comprises an expression element expressing following polypeptide (1) and polypeptide (2):
polypeptide (1): pelB signal peptide-variable region of the α chain-constant region of the α chain-Linker 1-A1 peptide;
polypeptide (2): gill signal peptide-variable region of the β chain-constant region of the β chain-Linker 2-B1 peptide-pIII protein;
preferably:
the amino acid sequence of the constant region of the α chain (C_{α}) is as shown in SEQ ID NO: 5;
and/or, the amino acid sequence of the constant region of the β chain (C_{β}) is as shown in SEQ ID NO: 4 or SEQ ID NO: 6;
and/or, the amino acid sequence of Linker 1 is as shown in SEQ ID NO: 3;
and/or, the amino acid sequence of Linker 2 is as shown in SEQ ID NO: 3;
and/or, the amino acid sequence of A1 peptide is as shown in SEQ ID NO: 1;
and/or, the amino acid sequence of B1 peptide is as shown in SEQ ID NO: 2;
and/or, the backbone of the phage vector is pCANTAB5E;
and/or, a ribosomal binding site and a molecular chaperone gene are integrated into the phage vector, such as, integrated at the 3' end of the gene expressing a capsid protein; the molecular chaperone gene is preferably the skp gene, more preferably is the sequence as shown in SEQ ID NO: 8; the sequence of the ribosomal binding site is preferably as shown in SEQ ID NO: 7;
and/or, the amino acid sequence of the constant region of the α chain is as shown in SEQ ID NO: 5;
and/or, the amino acid sequence of the constant region of the β chain is as shown in SEQ ID NO: 4 or SEQ ID NO: 6, or a variant thereof; the variant of the sequence as shown in SEQ ID NO: 6 preferably comprises mutation sites C75A and N89D.

9. A use of the polypeptide as shown in SEQ ID NO: 1 or 2 in screening a TCR mutant.

10. The use according to claim 9, wherein, during the use, the gene encoding the polypeptide is placed at the 3' end of the gene of constant region of the α chain or the β chain of the TCR, respectively;
preferably, the gene encoding the polypeptide is linked to the gene of the constant region of the α chain or the β chain of the TCR by a linker; the amino acid sequence of the linker is preferably as shown in SEQ ID NO: 3.
